# EUROPEAN PATENT SPECIFICATION

(11) **EP 1 341 904 B1**
(45) Date of publication and mention of the grant of the patent: **21.12.2005**
(21) Application number: 01980079.6
(22) Date of filing: 17.10.2001
(51) Int. Cl.: C12N 9/12

(54) **ZIPPER PROTEIN KINASE AND USES THEREOF**
ZIPPER PROTEINKINASE UND DEREN VERWENDUNGEN
PROTEINE KINASE ZIPPER ET SES UTILISATIONS

(30) Priority: 17.10.2000 US 240860 P
(43) Date of publication of application: 10.09.2003
(73) Proprietor: UNIVERSITE LAVAL, Québec, Québec G1K 7P4 (CA); Université de Sherbrooke, Sherbrooke, Québec J1K 2R1 (CA)
(72) Inventor: GERMAIN, Lucie, St-Augustin, Québec G3A 1H8 (CA); ROBITAILLE, Hubert, Charlesbourg, Québec G1H 6N6 (CA); FRADETTE, Julie, St-Damien de Bellechasse, Québec G0R 2Y0 (CA); BLOUIN, Richard, Sherbrooke, Québec J1E 3X4 (CA)
(74) Representative: Hinterberg, Katherine
(86) International application number: PCT/CA2001/001461
(87) International publication number: WO 2002/033064

(56) References cited:
- WO-A-00/08181
- WO-A-00/28324
- WO-A-99/09214
- US-A- 6 015 692
- ABE M K ET AL: "EXTRACELLULAR SIGNAL-REGULATED KINASE 7 (ERK7), A NOVEL ERK WITH A C-TERMINAL DOMAIN THAT REGULATES ITS ACTIVITY, ITS CELLULAR LOCALIZATION, AND CELL GROWTH" MOLECULAR AND CELLULAR BIOLOGY, WASHINGTON, DC, US, vol. 19, no. 2, February 1999 (1999-02), pages 1301-1312, XP002172155 ISSN: 0270-7306

## Description

### BACKGROUND OF THE INVENTION

### (a) Field of the Invention

The invention relates to the modulation of cell growth. Moat particularly, the invention relates to the use of protein kinase and its encoding gene to modulate the growth of different types of cells, as for example cancer cells.

### (b) Description of prior Art

A wide variety of circulating polypeptides and hormones play crucial roles in the regulation of growth, differentiation, and apoptosis in multicellular organisms. Many of these extracellular signaling molecules are transduced from the plasma membrane to the nucleus by a cascade of protein kinases that involves members of the mitogen-activated protein kinase (MAPK) family. The MAPK is classified into at least three distinct subfamilies of related polypeptides, ERK, JNK/SAPK and P38. Different types of extracellular stimuli preferentially activate each MAPK subgroup. In addition, the activation of each MAPK subgroup by different types of external stimuli requires phosphorylation of both a threonine and a tyrosine residue, and this is catalysed by specific MAPK kinases (MAPKK/MSK). The five members of the mixed lineage kinases family are all serine/threonine kinases and are involved in the MAPK signaling pathway and act as MAPKKK components.

WO 00/28324 relates to methods of modulating neuronal growth by modulating the p75NTR or MEK/MAPK signalling pathways.

WO 00/08181 describes the human Chp polypeptide, a cell signal transduction molecule which regulates PAK and JNK activity and interacts with the JNK MAP kinase cascade.

US 6,015,692 relates to the CDC37 cell-cycle regulatory protein which modulates cell-cycle progression.

WO 99/09214 describes compositions and methods to modulate Jun N-terminal kinase (JNK protein) and metastasis of malignant tumors.

Abe *et al.* (Molecular and Cellular Biology, Vol. 19, No.2, page 1301-1312, 1999) describe a member of the MAP kinase family termed extracellular signal-regulated kinase 7 (ERK7) which has the ability to function as a negative regulator of growth.

Serine/threonine kinase, such as leucine-zipper protein kinase (ZPK), are involved in the signaling pathways leading to the activation of various transcription factors. Some of these transcription factors are proto-oncogenes and may be implicated in the regulation of cell growth and differentiation.

Different kinases may be involved in cancer process or hyperproliferation. Regulation of cell growth is partly achieved through the activation of intracellular signaling pathways functioning both in a sequential and a parallel fashion. These signaling pathways regulate the critical phases of cell growth such as cell proliferation. Eukaryotic signaling pathways are typically arranged as networks of sequential protein kinases, which transmit the response to external stimuli, from the cell membrane to the nucleus. Aberrations in the function of protein kinases can lead to a spectrum of defects ranging from developmental abnormalities to numerous diseases, including cancer.

Absence or abnormalities in expression of such kinases participating in signal transduction may result in transformation of the cells and improved growth potential.

It would be highly desirable to be provided with new molecules and method for using it, enabling to treat or modulate the growth of cancer or normal cells.

### SUMMARY OF THE INVENTION

The present invention provides uses for the manufacture of a composition according to claims 1-12 and compositions according to claims 13-15 for inhibiting the growth of a cell or modulating the growth of a cell.

The cells targeted with the present invention may be a normal, a cancer cell, an epithelial cell, or a cell selected from the group consisting of a muscular cell, a neural cell, a fibroblast, and a mesenchymal cell.

The modulation of cell growth induced with the present invention may be a reduction or a suppression of the growth of said target cell.

The administration in a patient may be oral, intra-peritoneal, systemic, intravenous, cutaneous, transdermal, mucosal, rectal, or intratumoral administration, or topic application, or inhalation.

The nucleic acid sequence may be a ribonucleotide acid sequence or a deoxynucleotide acid sequence contained in an expression vector, the expression vector being a retrovirus, an adenovirus, a virus, a plasmid, a cosmid, an artificial chromosome, or a bacteriophage.

The expression vector may comprise a promoter which is ubiquitous or inducible.

Also, the expression vector can be further introduced into a cell before administration to a patient.

One object of the present invention is to provide the use of a MLK in the manufacture of a composition for the modulation of cell growth, wherein the MLK is selected from the group consisting of a leucine zipper kinase protein (ZPK), a dual leucine zipper bearing kinase (murine ZPK) or a mitogen-activated protein kinase-upstream kinase (MUK).

The cells can be in *in vitro* culture condition or in a patient.

In accordance with the present invention there is provided a MLK selected from the group consisting of a leucine zipper kinase protein (ZPK), a dual leucine zipper bearing kinase (murine ZPK) or a mitogen-activated protein kinase-upstream kinase (MUK) for inhibiting the growth of a cell.

Another object of the present invention is to provide a pharmaceutical composition comprising at least one MLK selected from the group consisting of a leucine zipper kinase protein (ZPK), a dual leucine zipper bearing kinase (murine ZPK) or a mitogen-activated protein kinase-upstream kinase (MUK) for inhibiting the growth of a cell.

Also, another object of the present invention is to provide at least one MLK selected from the group consisting of a leucine zipper kinase protein (ZPK), a dual leucine zipper bearing kinase (murine ZPK) or a mitogen-activated protein kinase-upstream kinase (MUK) for inhibiting the growth of a cell, wherein the MLK is under the form of a protein, a peptide, or a nucleic acid sequence encoding for said MLK.

For the purpose of the present invention the following terms are defined below.

There is provided by the present invention the use of a mixed lineage kinase (MLK), comprising the human variant leucine zipper protein kinase (ZPK), the murine variant dual leucine zipper bearing kinase (murine ZPK, or DLK) or the rat variant mitogen-activated protein kinase-upstream kinase (MUK), or their whole genes or portions of them, cDNA, mRNA, or nucleic acid sequence encoding for an efficient part thereof to reduce or suppress the growth of normal or cancer cells.

There is provided by the present invention the use of MLK protein or gene or efficient part of gene for cancer therapy or the treatment of various hyperproliferation diseases and/or cell differentiation dysfunctions.

The term "suppressing" is intended to mean reducing the frequency of tumor formation in individuals who are developing tumors or at risk of developing the same. A 2- to 10-fold reduction may be targeted, for example, in the percentage of at-risk individuals who form tumors after treatment with a MLK of cancer cells, relative to the percentage of at-risk individuals who form tumors when left untreated, may a target for treatment to be considered effective according to the invention. The term "suppressing" may be also intended to mean increasing the length of time required for tumor cells to arise after exposure to a tumorigenic substance (or, in the case of subjects with a genetic propensity to develop tumors, birth), reducing the rate of cell division in an existing tumor or causing regression (shrinkage in cell number) of an existing tumor. In order to be judged effective, an inhibition of cancer growth must lead to a reduction in the rate of cell division, and/or an increase in cell death.

The expressions "tumor cell" or "cancer cell" as used herein is intended to mean any cell that has undergone one or more rounds of cell division that take place outside the course of the growth of an organism to maturity, normal biological function or wound healing, characterized by a loss of contact inhibition or substrate dependence or changes in morphology and/or protein production.

As used herein, the term "malignant" refers to cancerous cell growth.

In contrast, "normal tissue" is defined as being one or more cells that are not tumor cells and/or that do not possess a malignant phenotype (that is, that do not display cancerous cell growth), as defined above.

As used herein to describe tumor cells, the term "growth" is defined as including induction (determination that a normal cell will become a tumor cell), transformation (differentiation of a normal cell to a tumor cell, whether or not such transformation is oncogenic) and proliferation.

The term " murine ZPK" or the expression "Dual leucine zipper bearing kinase" as used herein is intended to mean the murine variant of the human ZPK.

As used herein, the term "mammal" refers to any member of the Class Mammalia, including a human.

Preferably, the mammal is a human.

As used herein, the terms "reduction" or "suppression" are intended to mean a characteristic of the peptide or protein of the present invention which blocks or reverses the influx of cancer cells into tumor tissue or an adjacent site, whether directly or by inhibiting a signalling pathway that results in such infiltration. Such blocking or reversal may occur at the level of synthesis of a substance that promotes the maturation or migration of cancer cells.

Preferably the tumor comprises cells that are malignant.

The term "gene" as used herein is intended to mean a DNA sequence responsible for the production of a protein (or polypeptide). This includes first and foremost the actual coding sequence, which specifies the specific order of aminoacids in a polypeptide. In eukaryotes (animals with nucleated cells, such as mammals), that sequence may be distributed in short stretches of DNA called exons, interspersed with non-coding DNA sequences called introns. Those sequences, exons and introns, may be transcribed into a pre-messenger RNA, from which the introns may eventually be removed before the mature mRNA is translated into the actual protein encoded by the gene.

The term "promoter" as used herein is intended to mean non-coding regulatory sequences, usually located nearby the start of the coding sequence, which are referred to as the gene promoter. Put into a simplistic yet basically correct way, it is the interplay of the promoter with various specialised proteins called transcription factors that determines whether or not a given coding sequence may be transcribed and eventually translated into the actual protein encoded by the gene.

The term "regulatory element" as used herein refers to a DNA sequence that can increase or decrease the amount of product produced from another DNA sequence. The regulatory element can cause the constitutive production of the product (e.g., the product can be expressed constantly). Alternatively, the regulatory element can enhance or diminish the production of a recombinant product in an inducible fashion (e.g., the product can be expressed in response to a specific signal). The regulatory element can be controlled, for example, by nutrition, by light, or by adding a substance to a treated organism's system. Examples of regulatory elements well-known to those of ordinary skill in the art are promoters, enhancers, insulators, and repressors. A promoter typically increases the amount of recombinant product expressed from a DNA sequence as compared to the amount of the expressed recombinant product when no promoter exists. A promoter from one organism can be utilized to enhance recombinant product expression from a DNA sequence that originates from another organism. In addition, one promoter element can increase an amount of recombinant products expressed for multiple DNA sequences attached in tandem. Hence, one promoter element can enhance the expression of one or more recombinant products. Multiple promoter elements are well known to persons of ordinary skill in the art. Examples of promoter elements are described hereinafter.

The term "vector" as used herein refers to a nucleic acid, e.g., DNA derived from a plasmid, cosmid, virus, artificial chromosome or bacteriophage or synthesized by chemical or enzymatic means, into which one or more fragments of nucleic acid may be inserted or cloned which encode for particular genes. The vector can contain one or more unique restriction sites for this purpose, and may be capable of autonomous replication in a defined host or organism such that the cloned sequence is reproduced. The vector may have a linear, circular, or supercoiled configuration and may be complexed with other vectors or other material for certain purposes. The components of a vector can contain but is not limited to a DNA molecule incorporating DNA; a sequence encoding a excision protein or another desired product; and regulatory elements for transcription, translation, RNA stability and replication.

Any of the cell types defined herein can be altered to harbor modified nuclear DNA of the present invention.

Examples of methods for modifying a target DNA genome by insertion, deletion, and/or mutation are retroviral insertion, artificial chromosome techniques, gene insertion, random insertion with tissue specific promoters, homologous recombination, gene targeting, transposable elements, and/or any other method for introducing foreign DNA. Other modification techniques well known to a person of ordinary skill in the art include deleting DNA sequences, and/or altering nuclear DNA sequences.

The terms "transformation" and "transfection" as used herein refer to method of inserting foreign DNA into a cellular organism. These methods involve a variety of techniques, such as treating the cells with high concentrations of salt, an electric field, liposomes, polycationic micelles, or detergent, to render the host cell outer membrane or wall permeable to nucleic acid molecules of interest. Transfection techniques are well known to a person of ordinary skill in the art and materials and methods for carrying out transfection of DNA constructs into cells are commercially available. Materials typically used to transfect cells with DNA constructs are lipophilic compounds such as Lipofectin™. Particular lipophilic compounds can be induced to form liposomes for mediating transfection of the DNA construct into the cells. These specified methods are not limiting and the invention relates to any transfection technique well known to a person of ordinary skill in the art.

### BRIEF DESCRIPTION OF THE DRAWINGS

Fig. 1 illustrates an *in situ* hybridization of normal human skin sections with ZPK anti-sense probe and control sense probe;
Fig. 2 illustrates the detection of ZPK by western blot analysis;
Figs. 3A to 3L illustrate the detection of ZPK and involucrin by indirect immunofluorescence in normal human skin;
Figs. 4A to 4F illustrate the detection of ZPK and filaggrin in *in vitro* reconstructed skin;
Figs. 5A to 5H illustrate an immunofluorescence staining showing the ability of murine ZPK expression to arrest the growth of human epidermal cells (keratinocytes) and Figs. 5I to 5P illustrate an immunofluorescence staining showing the ability of murine ZPK expression to induce the differentiation of human epidermal cells (keratinocytes);
Fig. 6 illustrates a histogram showing the growth arrest of murine ZPK expressing keratinocytes;
Fig. 7 illustrates a histogram showing that murine ZPK induces keratinocytes differentiation;
Fig. 8 shows an immunofluorescence staining illustrating the growth arrest of murine ZPK expressing cancer cells; and
Fig. 9 shows an histogram illustrating the growth arrest of cancer cells.

### DETAILED DESCRIPTION OF THE INVENTION

It has been surprisingly discovered that administration, or exogenous expression of a certain quantity of at least one mixed lineage kinase (MLK) by cells induces a reduction, inhibition or suppression of the proliferation of these cells.

The leucine-zipper protein kinase (ZPK), the Dual leucine zipper bearing kinase (murine ZPK) and the MAPK upstream kinase (MUK) are members of the MLK of the present invention.

In accordance with one embodiment of the present invention, there is provided a method of reducing or suppressing the growth of cancer or normal cells by the administration into cells of a MLK under peptidic form or encoded into targeted cells by expression vectors. The cells can be of any type, as for example, but not limited to, epithelial cells.

The expression may be obtained by introducing a gene, a part of this gene or a cDNA coding for an active MLK into transfected or genetically transformed cells. Several types or forms of expression vectors can be employed to achieve this step of the invention.

The introduction into targeted cells of a gene, a portion of this gene or a cDNA coding for a MLK according to the invention can be made by transfection, infection or microinjection in the cells. Vectors can be introduced into host cells by, for example, the method using calcium phosphate, DEAE dextran, or cationic liposome DOTAP™ (Boehringer Mannheim), electroporation, or the calcium chloride method. The recombinant proteins can be purified from the obtained transformants.

Expression vectors may be a plasmid, a cosmid, a retrovirus, a virus, a bacteriophage, or any DNA construct that allows the expression of the MLK DNA sequence of the invention.

Furthermore, the DNAs of the present invention can be used for gene therapy of diseases caused by mutations in genomic DNAs. In gene therapy, the DNAs of the present invention may be administered to a living body inserted into adenovirus vectors, retrovirus vectors and so on. They can be administered by either ex *vivo* methods or *in vivo* methods.

In another embodiment of the present invention, there is provided a method using expression vectors in which the coding sequence may be under control of ubiquitous or inducible promoters or transcription regulatory elements.

The level of reduction of cell growth can be modulated depending of the expression level of MLK into a cell in which expression vectors have been introduced. This modulation can be influenced by the type of expression vector used as well as the number of vectors introduced into the targeted cells. Since the vector may contain a inducible promoter, the expression of the vector can be modulate proportionally to the treatment needed.

There is also provided with the present invention a method to stimulate or induce the expression or overexpression of MLK in epithelial cancer cells or in normal epidermal cells.

The normal or cancer cells that can be targeted with the present invention may be of epithelial origin, but they may also be from different other origins. For example, a targeted cell can be a fibroblast, a muscle cell, a nervous cell, an adipocyte, or other cell of mesenchymal origin.

One embodiment of the present invention provides a method of reducing or suppressing the growth of cancer cells at different levels of malignancy by administration of at least one MLK on or into targeted cells or by introduction of at least one expression vector coding for MLK.

According to one embodiment of the present invention, there is provided a method of diminishing, reducing, arresting or suppressing the growth of cells that may occur *in vitro* as well as *in vivo* or *in situ* conditions.

In another embodiment of the present invention, a MLK of the invention can be administered to an organism under the form of a peptide, or protein. The peptide, which encompasses the term protein, can be administered by different ways, as for exemple by oral administration, by injection in a pharmaceutical solution or carrier, or by topical application.

MLK under the form of a peptide is preferably the whole protein, as produced in nature. In certain cases, mostly for murine ZPK and MUK, it can be an active part of the protein.

In another embodiment of the present invention, there is also provided a method that may include the use of synthetic, semi-synthetic or recombinant forms of MLK, which can be introduced in cancer normal cells to reduce or suppress their growth.

In cases in which activity of the MLK is required at a site that is remote relative to the that of tumor cell induction or growth, systemic administration of a drug is generally appropriate. Methods of whole-body drug delivery are well known in the art. These include, but are not limited to, intravenous drip or injection subcutaneous, intramuscular, intraperitoneal, intracranial and spinal injection, ingestion via the oral route, inhalation, trans-epithelial diffusion (such as via a drug-impregnated, adhesive patch) or by the use of an implantable, time-release drug delivery device, which may comprise a reservoir of exogenously-produced MLK or may, instead, comprise cells that produce and secrete the MLK substance.

Alternatively, systemic administration is advantageous when MLK must be delivered to a target site that is accessible to topical application, but in which environment (such as the digestive tract) the native activity of the MLK might be compromised, e.g. by digestive enzymes or extremes of pH.

It is contemplated that global administration of the MLK to a human and an animal is not needed in order to achieve a highly localized effect. Given that an epithelial tumor is, by definition, on a surface of an organism, topical administration of a pharmaceutical composition is possible.

Topical compositions comprising an MLK can take any of several physical forms, or may consist in different delivery devices as summarized herein before; (i) A liquid, such as a tincture or lotion, which may be applied by pouring, dropping or "painting" (i. e. spreading manually or with a brush or other applicator such as a spatula); (ii) An ointment or cream, which may be spread either manually or with a brush or other applicator (e.g. a spatula or a patch), or may be extruded through a nozzle or other small opening from a container such as a collapsible tube; (iii) A dry powder, which may be shaken or sifted onto the site of potential or actual tumor cell growth or, alternatively, applied as a nebulized spray; (iv) A liquid-based aerosol, which may be dispensed from a container selected from the group that comprises pressure-driven spray bottles (such as are activated by squeezing), natural atomizers (or "pump-spray" bottles that work without a compressed propellant) or pressurized canisters; (v) A carbowax or glycerin preparation, such as a suppository, which may be used for rectal or vaginal administration of an MLK.

In a specialized instance, the internal surface is that of the lung. Epithelial tumors in the lung often result from long-term exposure to tobacco smoke or other chemical irritants. In such a case the most expedient route of administration for MLK is via inhalation, either of a liquid aerosol or of a nebulized powder. Drug delivery by inhalation, whether for topical or systemic distribution, is well known in the art for the treatment of asthma, bronchitis and anaphylaxis. In particular, it has been demonstrated that it is possible to deliver a protein via aerosol inhalation such that it retains its native activity in vivo (see Hubbard et al., 1989, J. Clin. Invest., 84: 1349-1354).

Note that in some cases, the internal surface in question may, for example, be found along the gastrointestinal tract lining; in such a case, topical application would comprise taking the drug via an oral route, whether in liquid, gel or solid form.

In the case of liquids, ointments and liquid-based aerosols, the preferred solvent is an aqueous medium with an ionic balance that mimics physiological salt levels in order to preserve activity of the MLK and to avoid changes in osmotic pressure for the cells to be contacted with the composition. An example of such medium is a low-ionic-strength saline solution.

A MLK influx into a site of tumor cell growth may comprise at least one MLK, carbohydrate or other biodegradable substance, therefore, depending upon the route of administration, it may be necessary to encapsulate or buffer it in such a way as to protect it from degradation (for example, by digestive enzymes, acid and base), at least until it reaches its target, by such methods as are well known in the pharmacological art.

The present invention will be more readily understood by referring to the following examples, which are given to illustrate the invention rather than to limit its scope.

### EXAMPLE I

### Localization of ZPK mRNA in human skin cells

### Materials and Methods

### Tissues and cell culture

The biopsies included newborn (24 h), children (2 and 4 yr.) foreskin, child finger (8 yr.), adult scalp (53 yr.) and trunk (16 and 25 yr.) skin. Specimens of skin samples and reconstructed skin were embedded in OCT compound (Miles Inc, Elkhart, IN), frozen in liquid nitrogen and stored at -70°C until use. Normal human keratinocytes (newborn foreskin, child finger) were isolated and cultured on a feeder layer of irradiated mouse 3T3 fibroblasts. Swiss 3T3 cells and adult human dermal fibroblasts were cultured in DME containing 10% FBS and antibiotics. Cultured cells were grown on glass coverslips, ethanol-fixed for 10 min at -20°C and processed for immunocytochemistry or grown on petri dishes for protein extraction.

### Immunohistochemical staining

Acetone-fixed frozen sections (4 µm-thick) of human tissues or ethanol-fixed cultured cells were analyzed following indirect immunofluorescence staining. The primary antibodies used include: a rabbit antiserum directed against the N-terminal portion (223 amino acids) of recombinant murine ZPK, and mouse monoclonal antibodies against involucrin (Sigma, Oakville, Canada), filaggrin and transglutaminase (BTI, Stoughton, MA) proteins. The goat secondary antibodies used were: TRITC conjugated anti-rabbit and anti-mouse (Chemicon™, Temecula, CA). Negative controls consisted of omission of the primary antibody during the labelling reaction. Cell nuclei were labeled with Hoechst reagent 33258 (Sigma).

### Protein extraction and immunoblotting

Swiss 3T3 and dermal human fibroblasts were cultured until confluence or 3 weeks after confluence, respectively. Cells in 100 mm petri dishes were lysed on ice in 1 ml of lysis buffer (phosphate-buffered saline (PBS) containing 1% Triton and protease suppressor cocktail (Roche Diagnostics, Laval, Canada)). Human epidermis was obtained from breast skin incubated in thermolysin solution (500 µg/ml in 10 mM Hepes and 1 mM CaCl₂) overnight at 4°C. Epidermis was separated from dermis with forceps, rinsed in PBS and cut in 1 ml of lysis buffer. All these extracts were incubated 30 min in lysis buffer at 4°C under agitation, and centrifuged 15 min at 12 000 X g. The concentration of total proteins in the supernatants was measured using the Micro BCA Protein Assay Reagent kit (Pierce, Rockford, Illinois). Proteins (35 µg) from cultured S3T3, cultured fibroblasts or fresh epidermis were separated under reducing conditions on a 7.5% SDS-polyacrylamide mini-gel. Proteins were transferred onto Hybond-C extra nitrocellulose membrane (Amersham Pharmacia Biotech Inc., Quebec, Canada) by electroblotting. Nitrocellulose membrane was blocked overnight at 4°C with 5% nonfat dry milk in a 10 mM Tris solution at pH 8.0 containing 0.9% NaCl and 0.05% Tween 20™ (TBST), and then incubated with ZPK antiserum (1/2000 in TBST, 1% milk) for 1 hr. After washing, the membrane was incubated with peroxidase-conjugated goat anti-rabbit IgG (Sigma, 1/4000) and detection done using the ECL system (Amersham) and Scientific Imaging Film (Kodak, Rochester, NY). In addition, adsorption of the rabbit antiserum has been done by incubation with recombinant glutathione S-transferase (GST)-ZPK coupled to glutathione sepharose beads (100 *µ*g), 2 hrs at 4°C. After centrifugation, the supernatant was used for Western blotting.

### Production of reconstructed skin in vitro

Reconstructed skin was produced as described before. Briefly, human fibroblasts were cultured for 35 days in medium supplemented with 50 *µ*g/ml of ascorbate (Sigma). The resulting sheets of cells and extracellular matrix were superimposed and formed the dermal equivalent on which human newborn foreskin keratinocytes were seeded. These reconstructed skins were cultured for 8 days in submerged conditions and then raised at the air-liquid interface for 21 days in order to achieve terminal differentiation.

### In situ RNA hybridization

*In situ* hybridization experiments were performed essentially as described previously using digoxigenin-label sense and antisense riboprobes generated from linearized pBluescript™ KS+ plasmid (Stratagene, CA) containing a 192 bp fragment of the murine ZPK cDNA. Immunological detection of the hybridized probes was performed with alkaline phosphatase-conjugated anti-digoxigenin antibody and nitroblue tetrazolium chloride/5-bromo-4-chloro-3-indolyl-phosphate color substrates (Roche Diagnostics, Laval, Canada).

### Murine ZPK overexpression in cultured keratinocytes: Cell proliferation assay

Human foreskin keratinocytes (8,000/cm²) were seeded with irradiated 3T3 fibroblasts (20,000/cm²) in 60 mm petri dishes and co-transfected after 2 d (40% confluence) with 0,5 µg of pEGFP-N1 plasmid (Clontech™, Palo Alto, CA) and 4, 5 µg of a pcDNA3™ (Invitrogen™) expression vector containing the entire coding region of murine ZPK cDNA (Douziech *et al*, 1999, J. Histo. Chemistry **47**:1287-1296). Keratinocytes transfected with 0,5 µg of pEGFP-N1 plasmid alone were used as controls. Transfection of the expression plasmids was done using Superfect™ Reagent (Qiagen, Missisauga, Ontario, Can) according to the manufacturer's protocol. After a 4-h incubation with the transfection mixture, the medium was replaced with 5 ml of fresh medium. Cells were then trypsinized 24 hours after and plated on glass coverslips (20, 000 cells/cm²). Cells were fixed with 4% formol-100% methanol 24 h, 48 h, 72 h, 96 h or 120 h after transfection (4 coverslips at each time points). Cells were immunostained by incubating with a rabbit anti-murine ZPK antiserum, followed by TRITC-conjugated goat anti-rabbit secondary antibody (Chemicon™, Temecula, CA), for 30 min. Negative controls and nuclei labelling were performed as described above. To evaluate if keratinocytes expressing murine ZPK had proliferated, the percentage of colonies containing 1 or more labeled cells was counted as a function of time after transfection. The experiment was performed twice.

### Murine ZPK overexpression in cultured keratinocytes: Filaggrin expression

Cells (Human foreskin keratinocytes) were transfected with 5 µg of either the murine ZPK pcDNA3 expression construct or the pEGFP-N1 plasmid as described above. Transfected cells were processed as mentioned before. Double indirect immunostaining was performed on ZPK transfected cells and non-transfected cells using, first, the rabbit anti-murine ZPK antiserum with TRITC-conjugated goat anti-rabbit secondary antibody (Chemicon™) and, second, a mouse anti-human filaggrin antibody (1/100 dilution) (BTI, Stoughton, MA) with a fluorescein-conjugated goat anti-mouse secondary antibody (Chemicon™). Immunostaining of pEGFP-N1 transfected control cells was performed using the mouse anti-human filaggrin antibody (BTI) and a TRITC-conjugated goat anti-mouse secondary antibody (Chemicon™). Cells were observed and counted by immunofluorescence microscopy. Cell nuclei were labeled with Hoechst reagent 33258 (Sigma).

### Murine ZPK overexpression in cultured epithelial cancer cells: proliferation assay

Cells (A-431, MCF-7 and SW-962)(ATCC) were seeded on glass coverslips (8,000/cm²) and cultured in DME containing 10% FBS and antibiotics until they reach 40-50% confluence (48-72h). Cells were transfected with 2 µg of a pcDNA3 (Invitrogen™) expression vector containing the entire coding region Murine ZPK cDNA (Douziech *et al*, 1999, J. Histo. Chemistry **47**:1287-1296). The transfection was done using Fugene 6™ reagent (Roche Diagnostics, Laval, Canada) according to the manufacturing protocol. After the transfection, the cells were grown 48 h, incubated with 5-bromodeoxyuridine (BrdU) for 2 h and 6 h and fixed with methanol 100%. Incorporation of BrdU in proliferating cells was detected by immunofluorescence using a DTAF labeled anti-BrdU antibody (Becton-Dickinson). Positive cells were observed and counted by immunofluorescence microscopy. The percentage of BrdU positive cells expressing ZPK was compared with the percentage of BrdU incorporation of the entire cell population.

### Results

### Localization of ZPK mRNA in human skin

Although the expression of ZPK mRNA has been demonstrated in human tissues such as brain, kidney, lung and skeletal muscle, little is known about this kinase. On the other hand, its murine variant murine ZPK is better characterized. For example, murine ZPK is expressed in post-mitotic cells of brain, and of various organs of foetal and adult mouse.

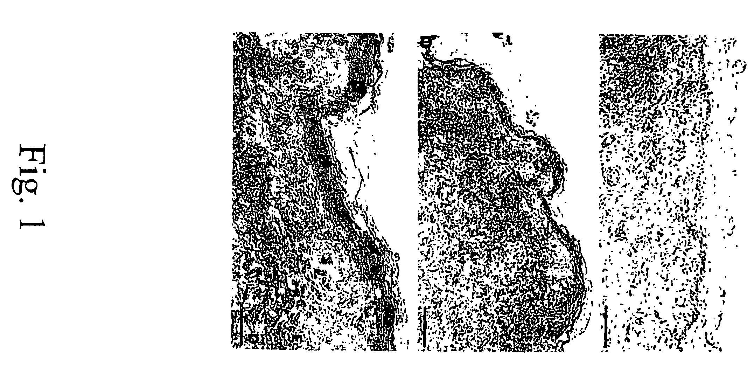

To examine the localization of ZPK mRNAs in human skin, *in situ* hybridization was performed with either antisense or sense digoxigenin-labeled murine ZPK probes on human newborn foreskin sections. As seen in Fig. 1b, ZPK mRNA was exclusively detected in the epidermal portion of the skin. Higher magnification (Fig. 1c) of the same section demonstrated that ZPK mRNA expression occurs specifically in keratinocytes of the granular layers of human epidermis. Control hybridization with the murine ZPK sense probe gave no signal (Fig. 1a). Similar localization in the granular layer of epidermis was observed for human adult trunk skin.

### ZPK protein is detected in extracts of human epidermis

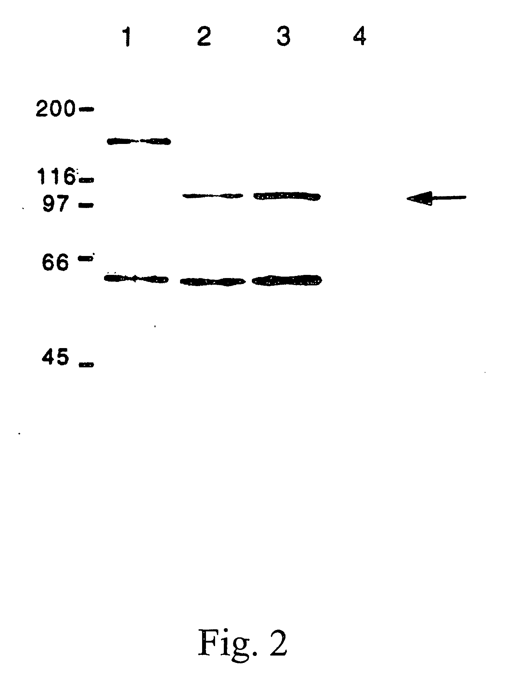

To monitor ZPK protein expression in human skin tissues, Western blot analysis were carried out using a rabbit antiserum raised against the N-terminal portion of recombinant murine ZPK. Fig. 2 showed the detection of human ZPK by western blot analysis of protein extracts from (1) cultured mouse S3T3 fibroblasts, (2) cultured human dermal fibroblasts, and (3,4) fresh human epidermis.

In human epidermis extracts (Fig. 2, *lane 3*), the antiserum recognized a protein with an apparent Mr of 104 000 that corresponds approximately to the molecular mass (94 kDa) predicted by its cDNA sequence (Reddy and Pleasure, 1994, Biochem. Biophys. Res. Commun. **202**:613). Therefore, this antiserum recognizes the human (Fig. 2, *lanes 2 and 3*) as well as murine (murine ZPK) (S3T3 extracts, Fig. 2, *lane 1*) variant. ZPK protein was also detected when long-term cultured human fibroblast extracts were analyzed (Fig. 2, *lane 2*). The detection of an additional band at 56 kDa for all the extracts is intriguing and to further test the specificity of the antibody, the rabbit antiserum was adsorbed to recombinant GST-murine ZPK protein coupled to glutathione sepharose beads. Western blotting with the adsorbed antiserum did not lead to the detection of any band (Fig. 2, *lane 4*). Reblotting of the same membrane with the non-adsorbed antiserum revealed the identical pattern of bands (Fig. 2, *lanes 1-3*) and led us to observe that the lower 56 kDa band represents a cleavage product of the ZPK protein. These results demonstrate that the antiserum was highly specific for human ZPK, and therefore suitable for immunohistochemical localization of the ZPK protein in human skin.

### ZPK protein localizes to keratinocytes of the granular layer of human epidermis and inner root sheath of hair follicles

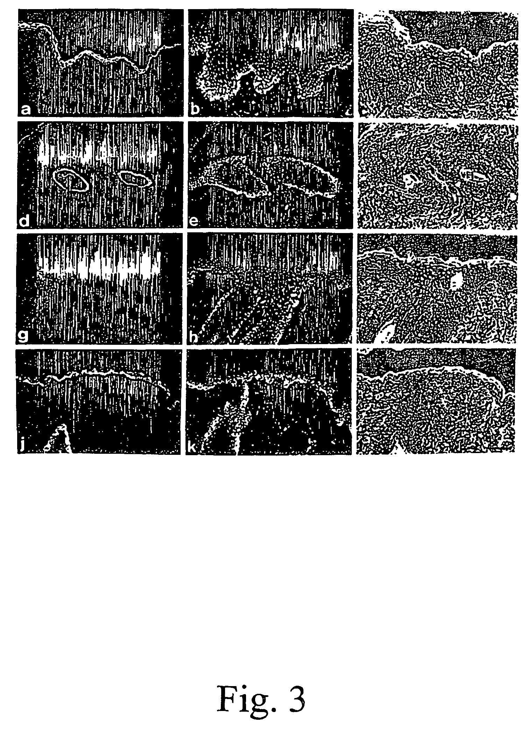

Skin biopsies from various body sites and different donor ages were examined to localize ZPK protein expression in normal human skin. Fig. 3 is indirect immunofluorescence labeling (a, d, g, i) of human adult scalp skin using (a,d) an anti-serum against ZPK protein showing reactivity within (a) epidermis and (d) hair follicles. (g) Negative control by omission of the primary antibody. (j) Labeling for involucrin, a protein expressed in the granular layer. (b, e, h, k) Nuclear Hoechst staining and (c, f, i, l) phase-contrast micrographs corresponding to (a), (d), (g), (j), respectively. Scale bar : 90 µm.

Staining of newborn foreskin, children finger skin, adult trunk and scalp skin frozen sections with anti-murine ZPK demonstrates that the protein was strongly expressed by the keratinocytes of the granular layer (Fig. 3a), the last nucleated layer of epidermis (Fig. 3b). When hair follicles were present, the IRS was strongly labeled (Fig. 3d). Some positive cells were also seen in sweat glands and at the openings of sebaceous glands on scalp skin sections. ZPK expression pattern to those of well-known differentiated-related proteins such as involucrin (Fig. 3j), filaggrin and transglutaminase was compared. ZPK localized to the same suprabasal compartments, namely the granular layer of epidermis and the inner root sheath of human hair follicles (Fig. 3).

The MLK, (ZPK, murine ZPK or MUK) protein and mRNA are localized in the very differentiated cells of the epidermis. Its localization supports that MLK is capable of playing a role in the regulation of the epidermal cells differentiation. In most cases, growth arrest occurs when cells start to differentiate. From this moment, many genes start to be expressed and that expression is often linked to the effect of transcription factors. Signaling pathways implicating many protein kinases activate such transcription factors. Kinases of the MLK family, such as ZPK, seem to participate to those signaling pathways.

### ZPK expression in cultured keratinocytes and reconstructed skin

In order to better define the distribution of ZPK in human keratinocytes, its expression pattern in cultured cells has been analyzed. For human keratinocytes, ZPK was not detected when these cells were cultured as monolayers with a feeder layer of irradiated 3T3 cells. In contrast, ZPK was detected in cultured human adult fibroblasts and NIH 3T3 cells (Fig. 2, *lanes 1, 2*). This might indicate a less restricted control of ZPK expression *in vitro* since no labelling of fibroblasts was seen in tissue sections (Fig. 3a).

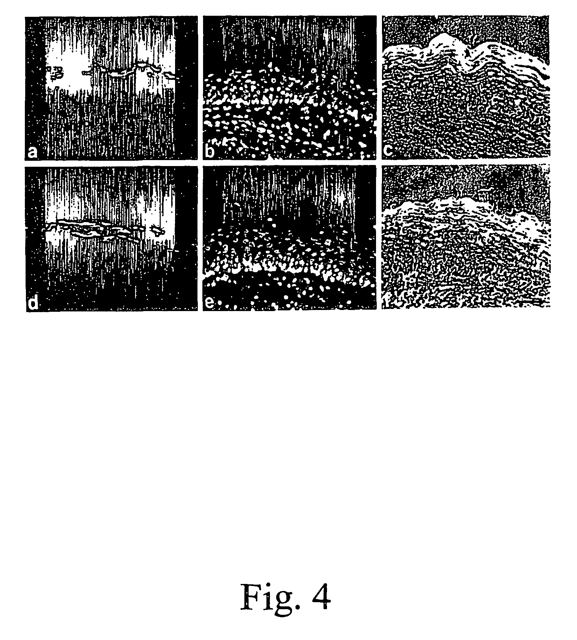

To test if ZPK expression could be found again in keratinocytes *in vitro* after induction of terminal differentiation, a reconstructed skin consisting of foreskin keratinocytes seeded on the top of a reconstructed dermis made up of human fibroblasts secreting their own matrix proteins was engineered. Fig. 4 is indirect immunofluorescence labelings of reconstructed human skin sections using (a) ZPK anti-serum and (d) an anti-filaggrin antibody. (b, e) Nuclear Hoechst stainings and (c, f) are corresponding phase-contrast micrographs of (a, d), respectively. Scale bar : 80 µm.

After 21 days of culture at the air-liquid interface, the reconstructed skin reached a high level of differentiation, as can be seen by the expression of filaggrin protein (Fig. 4d). In this reconstructed skin, ZPK expression was restored and localized to the upper suprabasal layers of epidermis. Even if ZPK expression was not as sharply defined in reconstructed skin (Fig. 4a) as a normal skin (Fig. 3a), it appears that the gene expression of ZPK is tightly controlled and differentiation-related in human keratinocytes under our culture conditions (Fig. 4).

### Murine ZPK overexpression in keratinocytes induces growth arrest and differentiation

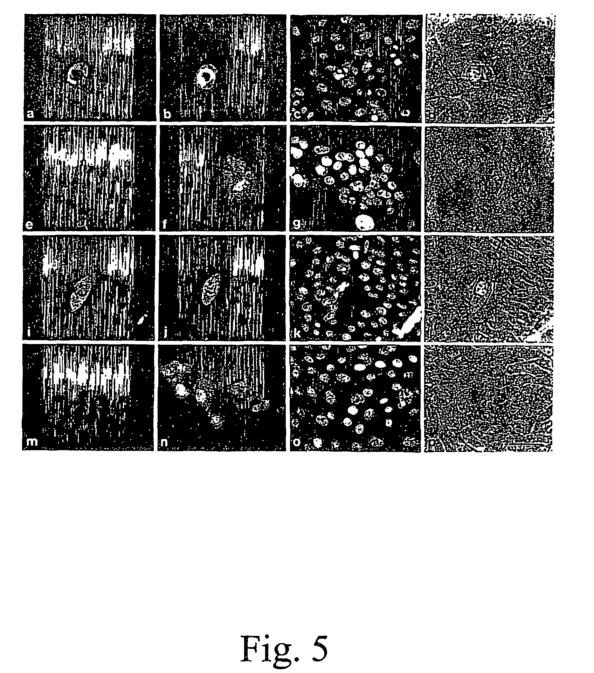

The advantage of the absence of ZPK in keratinocytes cultured in monolayer was utilised to evaluate the effect of its overexpression on keratinocyte proliferation and differentiation. In Fig. 5, cells were transfected with expression vectors for either murine ZPK and GFP (a-d), murine ZPK (i-1), or GFP (e-h, m-p) and labeled by indirect immunofluorescence using (a, e, i) murine ZPK anti-serum and (j, m) an anti-filaggrin antibody as described in *Materials and Methods.* (b, f, n) GFP fluorescence. (c, g, k, o) Nuclear Hoechst staining and (d, h, l, p) are corresponding phase-contrast micrographs of (a, e, i, m), respectively. Scale bar: 50 *µ*m.

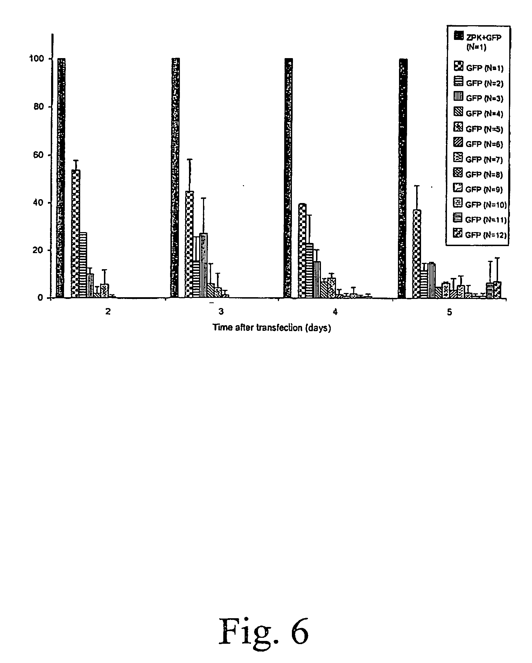

Fig. 6 is a histogram of keratinocytes transfected with expression vectors for murine ZPK and GFP (black column), or GFP alone (controls, white columns) as described in *materials and methods.* This figure represent the percentage of colonies containing N-labeled keratinocytes (N =1-12) on the total number of colonies containing labeled keratinocytes. Only one column is shown for ZPK-expressing keratinocytes as no colony containing more than one keratinocyte was observed. Values represent the mean ± SD of samples from two independent experiments.

The effect of murine ZPK expression on keratinocyte proliferation was determined by counting the number of ZPK-expressing cells present in each keratinocyte colony at different time after transfection with expression vectors for GFP and murine ZPK (Fig. 6). Cells expressing murine ZPK after transfection were always single in keratinocyte colonies indicating that they did not proliferate at all (Figs. 5a-d). In contrast, when GFP was expressed alone, the number of GFP expressing cells increased with time (Fig. 6). Two to twelve cells per colony were present in more than 60% of colonies containing GFP expressing cells 5 days after transfection indicating that GFP transfected keratinocytes had proliferated (Figs. 5e-h), Therefore, murine ZPK expression in keratinocytes induced a growth arrest.

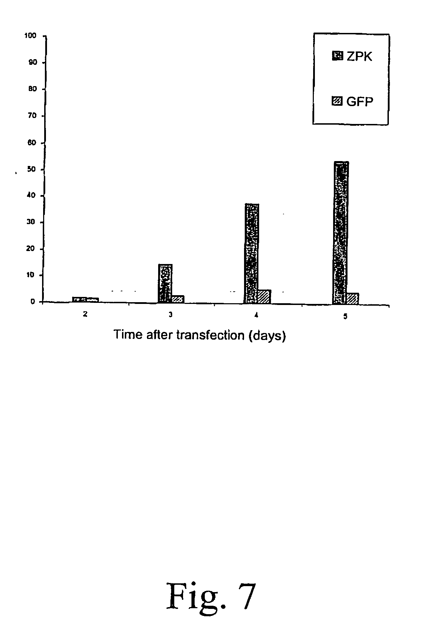

To evaluate if ZPK was involved in keratinocyte differentiation, the percentage of keratinocytes expressing filaggrin among the cells transfected with expression vectors for murine ZPK or GFP (control) was examined. Fig. 7 is a histogram of the percentage of keratinocytes transfected with murine ZPK (black column), or GFP (controls, white column) that coexpresses filaggrin.

The number of filaggrin expressing keratinocytes increased with time after transfection of the murine ZPK vector to reach 53% after 4 days (Fig. 7). Cells expressing murine ZPK and filaggrin were large and located on top of the colonies (Fig. 5i-l). In contrast, most of the GFP expressing keratinocytes were present in the basal layer of the colony. Moreover, only 4% of keratinocytes expressed filaggrin in these control cells (GFP). Therefore, it was observed that murine ZPK expression is sufficient to promote keratinocyte differentiation.

### Murine ZPK overexpression in cancer cells induces growth arrest

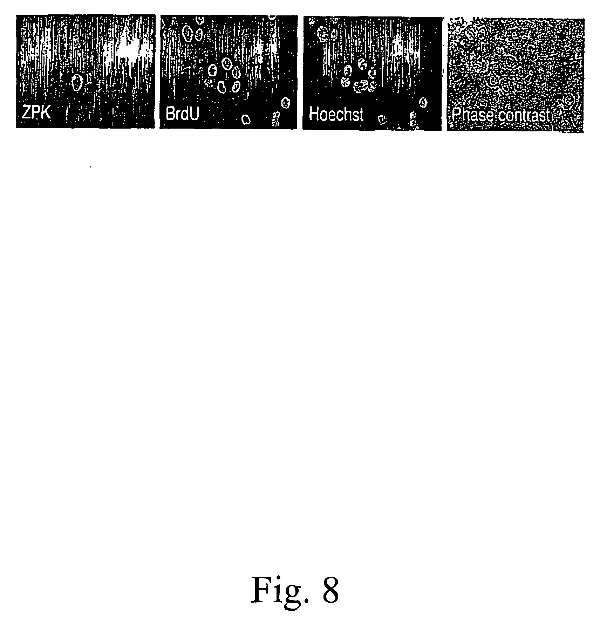

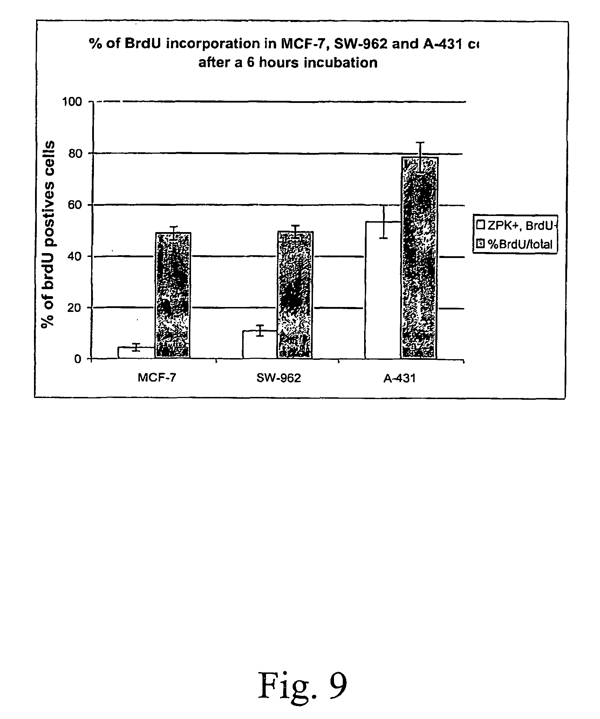

To evaluate the effect of MLK (ZPK, murine ZPK or MUK) overexpression on the three cancer cell lines (A-431, MCF-7 and SW-962), the rate of BrdU incorporation after a 2 h and a 6 h incubation of the murine ZPK expressing cells and the average of that BrdU incorporation in the whole population was compared. In normal culture conditions these three cell lines do not express or express a very low amount of ZPK protein. For A-431 cells, the BrdU incorporation showed about 20% less BrdU incorporation in murine ZPK expressing cells than in the entire cell population (Fig. 8). In the MCF-7 cell line the murine ZPK expressing cells showed a BrdU incorporation of less than 9% in comparison with more than 42% in the whole population at the two time points (Fig. 9). SW-962 cells expressing murine ZPK incorporated BrdU at a rate of about 10% after a 2 h incubation and 18% after a 6 h incubation and more than 50% in the untransfected cell population at the two time points (Fig. 9). These results demonstrated clearly that the expression of kinases of the MLK family, such as murine ZPK, induces a reduction or a suppression of growth of the epithelial cancer cells.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. Use of at least one MLK in the manufacture of a composition for the modulation of the growth of a target cell, said MLK being selected from the group consisting of a leucine zipper kinase protein (ZPK), a dual leucine zipper bearing kinase (murine ZPK), and a mitogen-activated protein kinase-upstream kinase (MUK).

2. The use of claim 1, wherein said cell is in *in vitro* culture condition or in a patient.

3. The use of claim 1, wherein said MLK is under the form of a protein, a peptide, or a nucleic acid sequence contained in an expression vector.

4. The use of claim 3, wherein said expression vector is a retrovirus, an adenovirus, a virus, a plasmid, a cosmid, an artificial chromosome, or a bacteriophage.

5. The use of claim 3, wherein said expression vector comprises a promoter which is ubiquitous or inducible.

6. The use of claim 3, wherein said nucleic acid sequence is a ribonucleotide acid sequence or a deoxynucleotide acid sequence.

7. The use of claim 1, wherein said modulation is a reduction or a suppression of the growth of said target cell.

8. The use of claim 3, wherein said expression vector is introduced into a cell before administration in a patient.

9. The use of claim 1, wherein said target cell is a normal or a cancer cell.

10. The use of claim 1, wherein said target cell is an epithelial cell.

11. The use of claim 1, wherein said target cell is selected from the group consisting of a muscular cell, a neural cell, a fibroblast, and a mesenchymal cell.

12. The use of claim 2, wherein said patient is a human or an animal.

13. A MLK selected from the group consisting of a leucine zipper kinase protein (ZPK), a dual leucine zipper bearing kinase (murine ZPK) or a mitogen-activated protein kinase-upstream kinase (MUK) for inhibiting the growth of a cell.

14. The MLK of claim 13, wherein said cell is in *in vitro* culture condition or in a patient.

15. A pharmaceutical composition comprising at least one MLK selected from the group consisting of a leucine zipper kinase protein (ZPK), a dual leucine zipper bearing kinase (murine ZPK) or a mitogen-activated protein kinase-upstream kinase (MUK) for modulating the growth of a cell.

## Patentansprüche

1. Verwendung von wenigstens einer MLK zur Herstellung einer Zusammensetzung für die Modulation des Wachstums von Zielzellen, wobei besagte MLK aus der Gruppe bestehend aus einer Leucinzipperkinase (ZPK), einer dualen Leucinzipper-tragenden Kinase (murine ZPK) und einer mitogen-aktivierten Proteinkinase - Upstreamkinase (MUK) ausgewählt ist.

2. Verwendung gemäß Anspruch 1, wobei besagte Zelle unter *in vitro* Kulturbedingungen oder in einem Patienten ist.

3. Die Verwendung gemäß Anspruch 1, wobei besagte MLK in Form eines Proteine, eines Peptids oder einer Nukleinsäuresequenz, die in einem Expressionsvektor enthalten ist, vorliegt.

4. Verwendung gemäß Anspruch 3, wobei besagter Expressionsvektor ein Retrovirus, ein Adenovirus, ein Virus, ein Plasmid, ein Kosmid, eine artifizielles Chromosom oder ein Bakteriophage ist.

5. Verwendung gemäß Anspruch 3, wobei besagter Expressionsvektor einen Promotor, der ubiquitar oder induzierbar ist, umfasst.

6. Verwendung gemäß Anspruch 3, wobei besagte Nukleinsäuresequenz eine Ribonukleinsäuresequenz oder eine Desoxynukleinsäuresequenz ist.

7. Verwendung gemäß Anspruch 1, wobei besagte Modulation eine Reduktion oder eine Unterdrückung des Wachstums besagter Zielzelle darstellt.

8. Verwendung gemäß Anspruch 3, wobei besagter Expressionsvektor in eine Zelle eingeführt wird bevor sie einem patienten verabreicht wird.

9. Verwendung gemäß Anspruch 1, wobei besagte Zielzelle eine normale Zelle oder eine Krebszelle ist.

10. Verwendung gemäß Anspruch 1, wobei besagte Zielzelle eine epitheliale Zelle ist.

11. Verwendung gemäß Anspruch 1, wobei besagte Zielzelle aus der Gruppe bestehend aus Muskelzellen, neuralen Zellen, Fibroblasten und mesenchymalen Zellen ausgewählt ist.

12. Verwendung gemäß Anspruch 2, wobei besagter Patient ein Mensch oder ein Tier ist.

13. Eine MLK ausgewählt aus der Gruppe bestehend aus einer Leucinzipper Kinase (ZPK), einer dualen Leucinzipper-tragenden Kinase (murine ZPK) oder einer mitogen-aktivierten Proteinkinase - Upstreamkinase (MUK) zur Hemmung des Wachstums einer Zelle.

14. Die MLK gemäß Anspruch 13, wobei besagte Zelle unter *in vitro* Kulturbedingungen oder in einem Patienten ist.

15. Eine pharmazeutische Zusammensetzung umfassend wenigstens eine MLK ausgewählt aus der Gruppe bestehend aus einer Leucinzipper Kinase (ZPK) einer dualen Leuinzipper-tragenden Kinase (murine ZPK) oder einer mitogen-aktivierten proteinkinase - Upstreamkinase (MUK) zur Modulation des Wachstums einer Zelle.

## Revendications

1. Utilisation d'au moins une MLK dans la fabrication d'une composition pour la modulation de la croissance d'une cellule cible, ladite MLK étant choisie parmi le groupe constitué de protéine kinase (ZPK), fermeture éclair de leucine, une kinase comportant une fermeture éclair de leucine mixte (murine ZPK), et une kinase en amont de la protéine kinase activée par des agents mitogènes (MUK).

2. L'utilisation selon la revendication 1, dans laquelle ladite cellule est en condition de culture *in vitro* ou chez un patient.

3. L'utilisation de la revendication 1, dans laquelle ladite MLK est sous forme de protéine, d'un peptide, ou d'une séquence d'acide nucléique présente dans un vecteur d'expression.

4. L'utilisation de la revendication 3, dans laquelle ledit vecteur d'expression est un rétrovirus, un adénovirus, un virus, un plasmide, un cosmide, un chromosome artificiel, ou un bactériophage.

5. L'utilisation de la revendication 3, dans laquelle ledit vecteur d'expression comprend un promoteur qui est omniprésent ou inductible.

6. L'utilisation de la revendication 3, dans laquelle ladite séquence d'acide nucléique est une séquence d'acide ribonucléotide ou une séquence d'acide deoxynucléotide.

7. L'utilisation de la revendication 1, dans laquelle ladite modulation est une réduction ou une suppression de la croissance de ladite cellule cible.

8. L'utilisation de la revendication 3, dans laquelle ledit vecteur d'expression est introduit dans la cellule avant administration chez un patient.

9. L'utilisation de la revendication 1, dans laquelle ladite cellule cible est une cellule normale ou cancéreuse.

10. L'utilisation de la revendication 1, dans laquelle ladite cellule cible est une cellule épithéliale.

11. L'utilisation de la revendication 1, dans laquelle ladite cellule cible est choisie parmi le groupe constitué d'un cellule de muscle, une cellule neurale, un fibroblaste, et une cellule mésenchimale.

12. L'utilisation selon la revendication 2, dans laquelle ledit patient est un humain ou un animal.

13. Une MLK choisie parmi le groupe constitué d'une protéine kinase fermeture éclair de leucine (ZPK), une kinase comportant une fermeture éclair de leucine mixte (murine ZPK), ou une kinase en amont de la protéine kinase activée par des agents mitogènes (MUK) pour prévenir la croissance d'une cellule.

14. La MLK de la revendication 13, dans laquelle ladite cellule est en condition de culture *in vitro* ou chez un patient.

15. Une composition pharmaceutique comprenant au moins une protéine kinase fermeture éclair de leucine (ZPK), une kinase comportant une fermeture éclair de leucine mixte (murine ZPK) ou une kinase en amont de la protéine kinase activée par des agents mitogènes (MUK), pour moduler la croissance d'une cellule.
